# EUROPEAN PATENT APPLICATION

(11) **EP 4 559 464 A1**
(43) Date of publication of application: **28.05.2025**
(21) Application number: 23842061.6
(22) Date of filing: 29.06.2023
(51) Int. Cl.: A61K 31/4706, A61P 19/02, A61P 37/02

(54) **USE OF NAPHTHOQUINE PHOSPHATE IN PREPARATION OF MEDICAMENT FOR TREATING AUTOIMMUNE DISEASES**

(30) Priority: 22.07.2022 CN 202210866097
(71) Applicant: Shanghai Pharmaceuticals Industry Co., Ltd, Shanghai 200002 (CN)
(72) Inventor: ZUO, Jianping, Shanghai 200002 (CN); MAO, Guanxing, Shanghai 200002 (CN); HE, Shijun, Shanghai 200002 (CN); ZHANG, Hongying, Shanghai 200002 (CN); WANG, Xue, Shanghai 200002 (CN); XING, Zhigang, Shanghai 200002 (CN); ZHAO, Limin, Shanghai 200002 (CN); DING, Xiying, Shanghai 200002 (CN)
(74) Representative: Nederlandsch Octrooibureau
(86) International application number: PCT/CN2023/103896
(87) International publication number: WO 2024/016996

(57) **Abstract**

Use of naphthoquine phosphate in preparation of a medicament for treating autoimmune diseases. It is proved in a mouse rheumatoid arthritis model that the intragastric administration of naphthoquine phosphate can remarkably ameliorate symptoms such as joint redness, swelling, and deformities and reduce the levels of antigen-specific antibodies. It is proved in a mouse systemic lupus erythematosus model that the intragastric administration of naphthoquine phosphate can remarkably reduce the levels of anti-double-stranded DNA antibodies and anti-nuclear antibodies. Pharmacodynamic and pharmacological studies prove that naphthoquine phosphate has ideal immunosuppressive activity and can be used to prepare the medicament for treating autoimmune diseases. Further provided is a pharmaceutical use of naphthoquine phosphate in the treatment and auxiliary treatment of rheumatoid arthritis or systemic lupus erythematosus. The medicament is a pharmaceutical composition consisting of a therapeutically effective amount of naphthoquine phosphate serving as an active ingredient and pharmaceutical excipients.

## Description

### TECHNICAL FIELD

The present invention relates to a medicament for use in new indications, specifically to the use of naphthoquine phosphate, which was used as an anti-malarial medicament, in preparation of a medicament for treating new indications, and in particular to the use of naphthoquine phosphate in preparation of a medicament for treating autoimmune diseases.

### BACKGROUND ART

Rheumatoid arthritis (RA) is a chronic, progressive, systemic and highly disabling autoimmune disease characterized by primary inflammatory synovitis, cartilage destruction and bone erosion. Common clinical symptoms are joint stiffness, swelling, and pain. As the disease progresses, it will cause destruction of joint cartilage and bone tissue, eventually leading to joint deformity and mobility impairment. The conditions of RA are complex. Clinically, medication remains the primary treatment approach, with the objectives of controlling disease symptoms, slowing disease progression, preventing bone joint damage, reducing disability rates, improving prognosis, and improving patients' quality of life (see literature [1]). Existing medicaments for treating RA mainly includes nonsteroidal anti-inflammatory medicaments, glucocorticoids, disease-ameliorating anti-rheumatic medicaments, biological preparations and small-molecule inhibitors. However, a considerable proportion of patients in clinical treatment exhibit low or no response to current treatments. Therefore, there is an urgent need to explore new treatments for RA.

Systemic lupus erythematosus (SLE) is a chronic autoimmune disease characterized by the formation of pathogenic autoantibodies and immune complexes, which mediate organ and tissue damage. Clinically, it manifests with multi-system involvement and the presence of a variety of autoantibodies in serum, primarily anti-nuclear antibodies. Due to the complexity of SLE pathogenesis, medicament development remains challenging. Currently, medicaments for treating SLE mainly include nonsteroidal anti-inflammatory medicaments, corticosteroids, anti-malarial medicaments, immunomodulators and biological preparations (see literature [2]).

Anti-malarial medicaments such as hydroxychloroquine and chloroquine are widely used in the clinical treatment of rheumatoid arthritis, systemic lupus erythematosus and other inflammatory rheumatic diseases. Hydroxychloroquine is currently the most commonly used anti-malarial medicament for treating autoimmune diseases. It reduces disease activity in patients with systemic lupus erythematosus, reduces the risk of organ damage and thrombosis, improves the condition of blood lipid, increases survival rates, improves metabolism of RA patients, and reduces the occurrence of cardiovascular events. ^{[3]}Naphthoquine phosphate, like quinine, chloroquine and hydroxychloroquine, is a quinoline derivative, and is suitable for the treatment of falciparum malaria. Naphthoquine phosphate has the following structural formula:

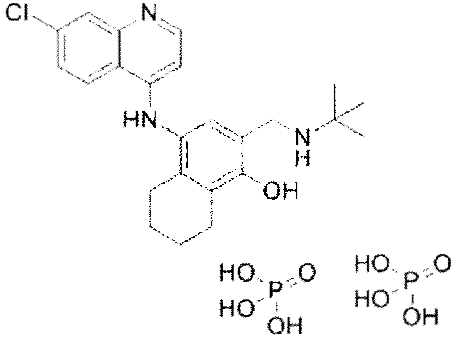

Currently, there are no known applications or reports on the use of naphthoquine phosphate for treating rheumatoid arthritis or systemic lupus erythematosus. The inventors utilized a collagen-induced arthritis mouse model and found that naphthoquine phosphate significantly improved clinical scores of arthritis in experimental mice, reduced levels of serum collagen-specific antibodies thereof, and inhibited lymphocyte proliferation. By using an adjuvant-induced arthritis rat model, it was found that the administration of naphthoquine phosphate significantly reduced clinical scores of arthritis in experimental rats and alleviated condition of foot swelling thereof. In an experimental mouse model of spontaneous systemic lupus erythematosus, intragastric administration of naphthoquine phosphate significantly reduced levels of anti-dsDNA antibodies and anti-nuclear antibodies in serum of the mouse model. These suggests that it could be developed as a medication for treating and auxiliary treating rheumatoid arthritis or systemic lupus erythematosus, which has great significance.

### SUMMARY OF THE INVENTION

The technical problem to be solved by the present invention is to further develop new uses of naphthoquine phosphate in medical science, in particular in preparation of a medicament for treating or auxiliary treating rheumatoid arthritis or systemic lupus erythematosus.

The present invention provides the use of naphthoquine phosphate in preparation of a medicament for treating an autoimmune disease.

The present invention has demonstrated that intragastric administration of naphthoquine phosphate can significantly improve symptoms such as joint redness, swelling and deformation, and reduce levels of antigen-specific antibodies in a rheumatoid arthritis mouse model. It has been demonstrated that intragastric administration of naphthoquine can significantly reduce levels of anti-double-stranded DNA antibodies and anti-nuclear antibodies in a systemic lupus erythematosus mouse model. Pharmacological studies have confirmed that naphthoquine phosphate has ideal immunosuppressive activity and can be used in preparation of a medicament for treating an autoimmune disease.

The present invention used a collagen-induced arthritis mouse model to demonstrate that naphthoquine phosphate can significantly improve clinical scores of arthritis in experimental mice, reduce levels of serum collagen-specific antibodies thereof, and inhibit lymphocyte proliferation, and used an adjuvant-induced arthritis rat model to demonstrate that administration of naphthoquine phosphate can significantly reduce clinical scores of arthritis in experimental rats and improve the condition of foot swelling thereof. In an experimental spontaneous systemic lupus erythematosus mouse model, intragastric administration of naphthoquine phosphate can significantly reduce levels of anti-double-stranded DNA antibodies and anti-nuclear antibodies in serum of the mouse model. These suggests that it can be developed as a medicament for treating and auxiliary treating rheumatoid arthritis or systemic lupus erythematosus.

The present invention significantly improves bovine type II collagen-induced arthritis in mice by intragastric administration of naphthoquine phosphate, demonstrating that naphthoquine phosphate can improve clinical scores of arthritis, reduce levels of pathogenic antibodies in serum, and can be used in preparation of a medicament for treating or auxiliary treating rheumatoid arthritis or systemic lupus erythematosus.

Therefore, the present invention also provides the use of naphthoquine phosphate in preparation of a medicament for improving arthritis disease indicators or reducing levels of lupus pathogenic antibodies.

The present invention provides the use of naphthoquine phosphate in preparation of a medicament for treating an autoimmune disease, wherein the medicament is a pharmaceutical composition consisting of a therapeutically effective amount of naphthoquine phosphate as an active ingredient and pharmaceutical excipient(s).

The present invention provides the use of naphthoquine phosphate in preparation of a medicament for treating or auxiliary treating rheumatoid arthritis or systemic lupus erythematosus.

The present invention provides the use of naphthoquine phosphate in preparation of a medicament for treating or auxiliary treating rheumatoid arthritis or systemic lupus erythematosus.

The medicament of the present invention is a pharmaceutical composition consisting of a therapeutically effective amount of naphthoquine phosphate as an active ingredient and pharmaceutical excipient(s).

The pharmaceutical composition is in a dosage form of a tablet, capsule, granule, oral liquid preparation, or in a dosage form of intravenous or intramuscular injection.

Furthermore, the present invention also provides a method for treating or auxiliary treating rheumatoid arthritis or systemic lupus erythematosus, including providing a therapeutically effective amount of naphthoquine phosphate to a subject in need thereof.

Furthermore, the present invention also provides a method for improving arthritis disease indicators or reducing levels of lupus pathogenic antibodies, including providing a therapeutically effective amount of naphthoquine phosphate to a subject in need thereof.

As used herein, the term "therapeutically effective amount" refers to an amount that has a therapeutic effect and is useful in preventing or treating a particular disease, disorder, or symptom described herein. For example, a "therapeutically effective amount" may refer to an amount necessary to provide a therapeutic or desired effect in a subject being treated. It is known by those skilled in the art that the therapeutically effective amount will vary depending on the route of administration, the use of excipients, and possibility of coadministration with other therapies.

The present invention provides the use of naphthoquine phosphate in preparation of a medicament for treating an autoimmune disease. By performing an experiment in a rheumatoid arthritis mouse model with naphthoquine phosphate, it has been demonstrated that intragastric administration of naphthoquine phosphate can significantly improve symptoms such as joint redness, swelling and deformation, and reduce levels of antigen-specific antibodies. It has been demonstrated that intragastric administration of naphthoquine can significantly reduce the levels of anti-double-stranded DNA antibodies and anti-nuclear antibodies in a systemic lupus erythematosus mouse model. Pharmacological studies have confirmed that naphthoquine phosphate has ideal immunosuppressive activity and can be used in preparation of a medicament for treating an autoimmune disease.

The present invention used a collagen-induced arthritis mouse model to demonstrate that naphthoquine phosphate can significantly improve clinical scores of arthritis in experimental mice, reduce levels of serum collagen-specific antibodies thereof, and inhibit lymphocyte proliferation, and used an adjuvant-induced arthritis rat model to demonstrate that administration of naphthoquine phosphate can significantly reduce clinical scores of arthritis in experimental rats and improve the condition of foot swelling thereof. In an experimental spontaneous systemic lupus erythematosus mouse model, intragastric administration of naphthoquine phosphate can significantly reduce levels of anti-dsDNA antibodies and anti-nuclear antibodies in serum of the mouse model. These further provides the pharmaceutical use of naphthoquine phosphate for treating and auxiliary treating rheumatoid arthritis or systemic lupus erythematosus. it has good clinical application prospects and great social benefits.

### BRIEF DESCRIPTION OF THE DRAWINGS

Figure 1 is a schematic diagram of results showing that intragastric administration of naphthoquine phosphate significantly improves clinical scores of bovine type II collagen induced-arthritis in mice.
Figure 2 is a schematic diagram of results showing that intragastric administration of naphthoquine phosphate inhibits bilateral hindfoot swelling in arthritic mice.
Figure 3 is a schematic diagram of results showing that intragastric administration of naphthoquine phosphate inhibits proliferation of lymphocytes in spleen and lymph nodes of arthritic mice.
Figure 4 is a schematic diagram of results showing that intragastric administration of naphthoquine phosphate reduces levels of collagen-specific antibodies in serum of arthritic mice.
Figure 5 is a representative picture showing that intragastric administration of naphthoquine phosphate improves foot swelling and suppresses bone erosion in arthritic mice.
Figure 6 is a schematic diagram of results showing that intragastric administration of naphthoquine phosphate can significantly inhibit activation of purified B cells mediated by Toll-like receptor signaling in spleen of arthritic mice.
Figure 7 is a schematic diagram of results showing that intragastric administration of naphthoquine phosphate significantly improves disease indicators of adjuvant-induced arthritis in rats.
Figure 8 is a schematic diagram of results showing that intragastric administration of naphthoquine phosphate significantly reduces levels of pathogenic antibodies in serum in experimental mice with spontaneous systemic lupus erythematosus.

### DETAILED DESCRIPTION OF THE EMBODIMENTS

Example 1. Inhibition of Mitogen-Induced Proliferation of Mouse Whole Spleen Lymphocytes by Naphthoquine Phosphate In Vitro

### 1. Main Experimental Materials and Sources

### (1) Experimental Animals

SPF-grade BALB/c mice, female, about 6-8 weeks old, purchased from Beijing HFK Bioscience Co., Ltd., with certificate number 110322211100631475.

### (2) Main Experimental Agents

Naphthoquine phosphate, light yellow powder, elemental analysis (C₂₄H₃₄ClN₃O₉P₂), supplied by Shanghai Pharmaceutical Industry Co., Ltd.; hydroxychloroquine sulfate, white powder, elemental analysis (C₁₈H₂₆ClN₃O·H₂SO₄), supplied by Zhongxi Sunve Pharmaceutical Co., Ltd. or purchased from Sigma; chloroquine, white powder, elemental analysis (C₁₈H₂₆CIN₃), purchased from Sigma.

### (3) Reagents

RPMI 1640 culture medium, purchased from GibcoBRL; fetal bovine serum, purchased from Hyclone; bacterial lipopolysaccharides (LPS), purchased from Sigma; concanavalin A (ConA), purchased from Sigma; hydroxychloroquine sulfate, supplied by Zhongxi Sunve Pharmaceutical Co., Ltd. or purchased from Sigma; thiazolyl blue tetrazolium bromide (MTT), purchased from Sigma; dimethyl sulfoxide (DMSO), purchased from Sinopharm; ³H-thymidine (³H-TdR), scintillation fluid, purchased from Perkin Elmer.

### 2. Experimental Methods

BALB/c mice were sacrificed by cervical dislocation, and spleens were aseptically excised to prepare whole spleen lymphocytes. The cells were adjusted to the required concentration. The compound concentrations started at 250 µM and was diluted with a 4-fold gradient to obtain 10 concentrations: 250, 62.5, 15.625, 3.906, 0.977, 0.244, 0.061, 0.015, 0.004 and 0.001 µM.

### (1) Non-Specific Cytotoxicity Assay

A suspension of mouse spleen lymphocytes was inoculated into a 96-well plate at 8×10⁵/well, and the above 10 concentration gradients of the compound were added. A corresponding cell control (i.e. spleen lymphocyte culture system without compound) and a RPMI 1640 culture medium background control (blank culture medium control) were set up. The cells were cultured in a 37°C, 5% CO₂ incubator for 48 hours. Four hours before the end of the culture, 20 µl of 5 mg/ml MTT solution was added, and the reaction continued until the end of the culture (i.e. the aforementioned 48 hours). The supernatant was aspirated and discarded, 200 µl DMSO was added to each well to dissolve the crystals. A suspension of mouse spleen lymphocytes was obtained. The absorbance value was measured at 570 nM with a microplate reader (Spectra Max 190, Molecular Devices).

### (2) Lymphocyte Proliferation Assay

A suspension of mouse spleen lymphocytes was inoculated into a 96-well plate at 5×10⁵/well, and ConA (final concentration: 1 µg/ml) or LPS (final concentration: 10 µg/ml) and the above 10 concentration gradients of the compound were added. Corresponding cell control wells without ConA and LPS, as well as medicament-free stimulated control wells, were set up. The cells were cultured in a 37°C, 5% CO₂ incubator for 48 hours. Eight hours before the end of the culture, ³H-thymidine was added, and the culture continued until the end of the experiment (i.e., the aforementioned 48 hours). The cells were collected onto glass fiber membranes using a cell collector. 5 ml scintillation fluid was added, and the values of radioactive counts per minute were read on a Beta counter (2450 Microplate Counter, PerkinElmer) (model).

### 3. Experimental Results

ConA and LPS, as mitogens, stimulated proliferation and differentiation of T and B lymphocytes, respectively. This process is similar to the activation process of lymphocytes *in vivo.* Therefore, mitogen-induced lymphocyte proliferation is commonly used as an indicator to evaluate functionality of lymphocytes. As shown in Table 1, naphthoquine phosphate has significant inhibitory activity on ConA-induced T cell activation and proliferation, as well as on LPS-induced B cell activation and proliferation. The biological activity selection index (SI) of naphthoquine phosphate was superior to that of chloroquine and hydroxychloroquine.

**Table 1. Inhibition of mitogen-induced proliferation of mouse whole spleen lymphocytes by naphthoquine phosphate in vitro**

| Compound | CC50(µM) | ConA | | LPS | |
|---|---|---|---|---|---|
| | | IC50 (µM) | SI | IC50 (µM) | SI |
| Naphthoquine Phosphate | 1.45 | 0.41 | 3.5 | 0.08 | 17.71 |
| Chloroquine Phosphate | 26.94 | 14.33 | 1.9 | 15.08 | 1.79 |
| Hydroxychloroquine Sulfate (Sigma) | 25.16 | 17.08 | 1.5 | 14.88 | 1.69 |
| Hydroxychloroquine Sulfate (Zhongxi Sunve) | 25.15 | 17.02 | 1.5 | 14.89 | 1.69 |
| CsA | 5.56 | 0.01 | 500.2 | 0.08 | 68.91 |

| | | | | | |
|---|---|---|---|---|---|
| Note: CC50 is the compound concentration at which 50% of the cells survive; IC50 is the compound concentration at which cell proliferation is reduced by 50%; SI = CC50/IC50. | | | | | |

The above results show that naphthoquine phosphate can significantly inhibit mitogen-induced activation of spleen lymphocytes, especially its inhibitory activity on B cell activation and proliferation. Additionally, its immunosuppressive activity was higher than that of chloroquine and hydroxychloroquine sulfate.

### Example 2. Inhibition of LPS-Induced Macrophage Inflammatory Cytokine Production by Naphthoquine Phosphate

### 1. Main Experimental Materials and Sources

### (1) Cells

Mouse macrophage cell line RAW264.7, purchased from American Type Culture Collection (ATCC).

### (2) Main Experimental Agents

Naphthoquine phosphate, light yellow powder, elemental analysis (C₂₄H₃₄ClN₃O₉P₂), supplied by Shanghai Pharmaceutical Industry Co., Ltd.; hydroxychloroquine sulfate, white powder, elemental analysis (C₁₈H₂₆ClN₃O·H₂SO₄), supplied by Zhongxi Sunve Pharmaceutical Co., Ltd.; chloroquine, white powder, elemental analysis (C₁₈H₂₆CIN₃), purchased from Sigma.

### (3) Reagents

DMEM high-glucose medium, purchased from GibcoBRL; fetal bovine serum, purchased from Hyclone; LPS, purchased from Sigma-Aldrich; TNF-α and IL-6 ELISA kits, purchased from BD, USA; mouse IL-1β cytokine ELISA detection kit, purchased from Invitrogen; MTT, purchased from Sigma; DMSO, purchased from Sinopharm.

### 2. Experimental Methods

### (1) Non-Specific Cytotoxicity Assay

RAW264.7 cells were inoculated into a 96-well plate at 1×10⁵/well and allowed to adhere to the wall for 6 hours in a 37°C, 5% CO₂ incubator. Compounds were diluted with a 4-fold gradient into 10 concentrations starting from 250 µM. After the cells adhered to the wall, gradient diluted compounds (compound concentration was diluted with a 4-fold gradient into 10 concentrations starting from 250 µM: 250, 62.5, 15.625, 3.906, 0.977, 0.244, 0.061, 0.015, 0.004 and 0.001 µM) were added. A corresponding solvent control (cell control) and a culture medium background control (blank control) were set up. The cells were cultured in a 37°C, 5% CO₂ incubator for 48 hours. Fifteen minutes before the end of the culture, 20 µl of 5 mg/ml MTT was added. At the end of the culture, the supernatant was aspirated and discarded, 200 µl DMSO was added to dissolve the crystals. The absorbance value was measured at 570 nM with a microplate reader (Spectra Max 190, Molecular Devices).

### (2) Cytokine Production Level Detection

RAW264.7 cells were inoculated into a 96-well plate at 1×10⁵/well and allowed to adhere to the wall for 6 hours in a 37°C, 5% CO₂ incubator. Compounds were diluted with a 4-fold gradient into 10 concentrations starting from 250 µM: 250, 62.5, 15.625, 3.906, 0.977, 0.244, 0.061, 0.015, 0.004 and 0.001 µM. After the cells adhered to the wall, the stimulant LPS (final concentration: 10 µg/ml) and the gradient-diluted compounds were added. A corresponding stimulation control and a cell control (without stimulation) were set up. After the end of the culture, the cell culture supernatants were collected, and the levels of TNF-α, IL-1β and IL-6 were measured by ELISA.

### 3. Experimental Results

LPS is an important biological pro-inflammatory factor that induces the release of a large number of inflammatory cytokines by activating nuclear factor kappa-B (NF-κB). Therefore, an LPS-induced macrophage RAW264.7 system was used to evaluate the effects of the compounds on the production of inflammatory cytokines. As shown in Table 2, naphthoquine phosphate, hydroxychloroquine sulfate and chloroquine all significantly inhibited the production of LPS-induced IL-1β, but had no significant effect on the production of TNF-α and IL-6.

**Table 2. Effects of naphthoquine phosphate on the production of inflammatory cytokines by LPS-induced RAW264.7 cells**

| Compound | CC₅₀(µM) | TNF-α | | IL- 1β | | IL-6 | |
|---|---|---|---|---|---|---|---|
| | | IC₅₀(µM) | SI | IC₅₀(µM) | SI | IC₅₀(µM) | SI |
| Naphthoquine Phosphate | 8.73 | >10 | \ | 1.59 | 5.48 | >10 | \ |
| Hydroxychloroquine Sulfate | 24.35 | 46.30 | 0.5 | 3.5 | 6.96 | 16.53 | 1.47 |
| Chloroquine Sulfate | 26.64 | 32.62 | 0.8 | 2.72 | 9.81 | 14.71 | 1.81 |

| | | | | | | | |
|---|---|---|---|---|---|---|---|
| Note: CC50 is the compound concentration at which 50% of the cells survive; IC50 is the compound concentration at which cytokine secretion is reduced by 50%; SI = CC50/IC50. | | | | | | | |

The above results show that naphthoquine phosphate selectively inhibits the secretion of inflammatory cytokines in LPS-induced mouse macrophage cell line RAW264.7 cells.

Example 3. Inhibition of TLRs-L-Stimulated Proliferation of Mouse Whole Spleen Lymphocytes and Secretion of Antibodies and Cytokines by Naphthoquine Phosphate In Vitro

### 1. Main Experimental Materials and Sources

### (1) Experimental Animals

SPF-grade BALB/C mice, female, about 6-8 weeks old, purchased from Beijing HFK Bioscience Co., Ltd., with certificate number 110322211100631475.

### (2) Main Experimental Agents

Naphthoquine phosphate, light yellow powder, elemental analysis (C₂₄H₃₄ClN₃O₉P₂), supplied by Shanghai Pharmaceutical Industry Co., Ltd.; hydroxychloroquine sulfate, white powder, elemental analysis (C₁₈H₂₆ClN₃O·H₂SO₄), supplied by Zhongxi Sunve Pharmaceutical Co., Ltd.

### (3) Reagents

RPMI 1640 culture medium, purchased from GibcoBRL; fetal bovine serum, purchased from Hyclone; TLR 4, 7, 9 agonists (TLRs Ligand, TLRs-L), purchased from Invivogen; mouse IL-6, TNF-α, IL-10 cytokine ELISA detection kit, purchased from BD; mouse IL-1β cytokine ELISA detection kit, purchased from Invitrogen; antibody detection kit, purchased from Invitrogen.

### 2. Experimental Methods

BALB/c mice were sacrificed by cervical dislocation, and spleens were aseptically excised to prepare whole spleen lymphocytes. The cells were adjusted to 5×10⁶ cells/ml. Hydroxychloroquine sulfate was diluted with a 3-fold gradient into 3 concentrations starting from 30 µM: 30, 10 and 3 µM; naphthoquine phosphate was diluted with a 3-fold gradient into 3 concentrations starting from 10 µM: 10, 3 and 1 µM.

### (1) Lymphocyte Proliferation Assay

A suspension of mouse spleen lymphocytes was inoculated into a 96-well plate at 100 µl/well, and the stimulant TLR4-L (final concentration: 10 µg/ml), TLR7-L (final concentration: 5 µg/ml) or TLR9-L (final concentration: 1 µM) and compounds diluted with the above concentration gradients were added. A corresponding stimulation control and a cell control (without stimulation) were set up. The cells were cultured in a 37°C, 5% CO₂ incubator for 48 hours. Eight hours before the end of culture, ³H-TdR was added, and the culture continued until the end of the experiment. The cells were collected onto glass fiber membranes using a cell collector. 5 ml scintillation fluid was added, and the values of radioactive counts per minute were read on a Beta counter (2450 Microplate Counter, PerkinElmer).

### (2) Cytokine Detection

Mouse spleen lymphocytes were inoculated into a 96-well plate at 100 µl/well, and the stimulant TLR4-L (final concentration: 10 µg/ml), TLR7-L (final concentration: 5 µg/ml) or TLR9-L (final concentration: 1 µM) and the compounds diluted with the above concentration gradients were added. A corresponding stimulation control and a cell control (without stimulation) were set up. The cells were cultured in a 37°C, 5% CO₂ incubator for 48 hours. After the end of the culture, the cell culture supernatants were collected, and the levels of IL-1β, IL-6, and IL-10 were measured by ELISA.

### (3) Antibody Level Detection

Mouse spleen lymphocytes were inoculated into a 96-well plate at 100 µl/well, and the stimulant TLR4-L (final concentration: 10 µg/ml), TLR7-L (final concentration: 5 µg/ml) or TLR9-L (final concentration: 1 µM) and the compounds diluted with the above concentration gradients were added. A corresponding stimulation control and a cell control (without stimulation) were set up. The cells were cultured in a 37°C, 5% CO₂ incubator for 120 hours. After the end of the culture, the cell culture supernatants were collected, and the levels of IgG and IgM were measured by ELISA.

### 3. Experimental Results

Toll-like receptors (TLRs) belong to the pattern recognition receptor family and are expressed in a variety of immune cells. They recognize a variety of pathogen-related molecular patterns and mediate the differentiation and maturation of antigen-presenting cells. They play a critical role in inflammation, regulation, survival and proliferation of immune cells. As shown in Tables 3 and 4, TLRs-L mediated the proliferation and activation of spleen lymphocytes. The effects of naphthoquine phosphate significantly inhibited the proliferation and cytokine and antibody secretion mediated by specific TLRs-L.

**Table 3. Effects of naphthoquine phosphate on the proliferation of mouse whole spleen lymphocytes and its antibody secretion stimulated by TLRs-L**

| Compound | CC₅₀ (µM) | Stimulant | Proliferation | | IgG | | IgM | |
|---|---|---|---|---|---|---|---|---|
| | | | IC₅₀ (µM) | SI | IC₅₀ (µM) | SI | IC₅₀ (µM) | SI |
| Hydroxychloroquine Sulfate | 26.95 | TLR4-L | 6.607 | 4.079 | 18.190 | 1.482 | 25.280 | 1.066 |
| | | TLR7-L | 18.950 | 1.422 | 26.900 | 1.002 | 19.220 | 1.402 |
| | | TLR9-L | 8.790 | 3.066 | 5.626 | 4.790 | 2.506 | 10.754 |
| Naphthoquine Phosphate | 1.59 | TLR4-L | 0.129 | 12.316 | \ | \ | 0.845 | 1.881 |
| | | TLR7-L | 1.051 | 1.513 | \ | \ | \ | \ |
| | | TLR9-L | 0.218 | 7.304 | 0.325 | 4.888 | 2.027 | 0.84 |

| | | | | | | | | |
|---|---|---|---|---|---|---|---|---|
| Note: CC50 is the compound concentration at which 50% of the cells survive; IC50 is the compound concentration at which cell proliferation and antibody secretion are reduced by 50%; SI=CC50/IC50. | | | | | | | | |

**Table 4. Effects of naphthoquine phosphate on cytokine secretion by mouse whole spleen lymphocytes stimulated by TLRs-L**

| Compound | CC₅₀ (µM) | Stimulant | IL-1β | | IL-6 | | IL-10 | |
|---|---|---|---|---|---|---|---|---|
| | | | IC₅₀(µM) | SI | IC₅₀(µM) | SI | IC₅₀(µM) | SI |
| Hydroxychloroquine Sulfate | 26.95 | TLR4-L | 31.970 | 0.843 | 15.320 | 1.759 | 3.188 | 8.454 |
| | | TLR7-L | \ | \ | 1.905 | 14.147 | 1.108 | 24.323 |
| | | TLR9-L | 41.62 | 0.648 | 3.924 | 6.868 | 3.296 | 8.177 |
| Naphthoquine Phosphate | 1.59 | TLR4-L | 3.349 | 0.475 | 0.630 | 2.524 | 0.106 | 14.972 |
| | | TLR7-L | \ | \ | 0.007 | 233.824 | 0.039 | 40.769 |
| | | TLR9-L | \ | \ | 0.290 | 5.483 | 0.190 | 8.364 |

| | | | | | | | | |
|---|---|---|---|---|---|---|---|---|
| Note: CC50 is the compound concentration at which 50% of the cells survive; IC50 is the compound concentration at which cytokine secretion is reduced by 50%; SI = CC50/IC50. | | | | | | | | |

The above results indicate that naphthoquine phosphate can inhibit the activation of spleen lymphocytes induced by specific TLR signaling.

Example 4. Inhibition of TLR Agonists-Stimulated Activation of Purified Mouse B Cells by Naphthoquine Phosphate In Vitro

### 1. Main Experimental Materials and Sources

### (1) Experimental Animals

SPF-grade BALB/C mice, female, about 6-8 weeks old, purchased from Beijing HFK Bioscience Co., Ltd., with certificate number 110322211102061763.

### (2) Main Experimental Agents

Naphthoquine phosphate, light yellow powder, elemental analysis (C₂₄H₃₄ClN₃O₉P₂), supplied by Shanghai Pharmaceutical Industry Co., Ltd.; hydroxychloroquine sulfate, white powder, elemental analysis (C₁₈H₂₆ClN₃O·H₂SO₄), supplied by Zhongxi Sunve Pharmaceutical.

### (3) Reagents

RPMI 1640 culture medium, purchased from GibcoBRL; fetal bovine serum, purchased from Hyclone; PE-CD19: purchased from BD; Anti-PE Beads: purchased from Miltenyi; TLRs-L, purchased from Invivogen; mouse IL-6 antibody detection kit, purchased from Invitrogen; MTT, purchased from Sigma; and DMSO, purchased from Sinopharm.

### 2. Experimental Methods

### (1) Preparation of Purified B Cells

BALB/c mice were sacrificed by cervical dislocation, and spleens were aseptically excised to prepare spleen lymphocytes. The cells were adjusted to 1×10⁸ cells/ml, blocked with 2.4G2 (final concentration: 10 µg/ml) for 20 min, cultured with PE-CD19 (final concentration: 5 µg/ml) for 20 min, washed with MACS buffer, centrifuged at 4°C, 300G for 10 min, 100µl anti-PE beads were added per 1×10⁸ cells, incubated with 900 µl MACS buffer for 15 min, washed with MACS buffer, centrifuged at 4°C, 300G for 10 min. The supernatant was discarded. 500 µl MACS buffer was added per 1×10⁸ cells to resuspend the cells. MACS column was used for separation. Magnetically labeled cells were retained on the column. The MACS column was removed from the magnetic field and then eluted.

### (2) Non-Specific Cytotoxicity Assay

Hydroxychloroquine sulfate was diluted with a 2-fold gradient into 3 concentrations starting from 8 µM: 8, 4 and 2 µM. Naphthoquine phosphate was diluted with a 2-fold gradient into 3 concentrations starting from 0.5 µM: 0.5, 0.25, 0.125 µM. Purified mouse B cells were inoculated into a 96-well plate at 1×10⁶/well, and gradient diluted compounds were added. A corresponding blank control and a cell control were set up. The cells were cultured in a 37°C, 5% CO₂ incubator for 48 hours. Four hours before the end of the culture, MTT solution was added. At the end of the culture, the supernatant was aspirated and discarded, 200 µl DMSO was added to each well to dissolve the crystals. The absorbance value was measured at 570 nm with a microplate reader (Spectra Max 190, Molecular Devices).

### (3) B Cell Proliferation Assay

Hydroxychloroquine sulfate was diluted 2-fold into 3 concentrations starting from 8 µM: 8, 4 and 2 µM. Naphthoquine phosphate was diluted 2-fold into 3 concentrations starting from 0.5: 0.5, 0.25 and 0.125 µM. A suspension of purified mouse B cells was inoculated into a 96-well plate at 4×10⁵/well, and the stimulant TLR4-L (final concentration: 10 µg/ml), TLR7-L (final concentration: 5 µg/ml) or TLR9-L (final concentration: 1 µM) and the gradient diluted compounds were added. A corresponding stimulation control and a cell control (without stimulation) were set up. The cells were cultured in 37°C, 5% CO₂ incubator for 48 hours. Eight hours before the end of the culture, ³H-TdR was added, and the culture was continued until the end of the experiment. The cells were collected onto glass fiber membranes using a cell collector. 5 ml scintillation fluid was added, and the values of radioactive counts per minute were read on a Beta counter (2450 Microplate Counter, PerkinElmer).

### (4) Cytokine Detection

A suspension of purified mouse B cells was inoculated into a 96-well plate at 4×10⁵/well. Hydroxychloroquine sulfate was diluted with a 2-fold gradient into 3 concentrations starting from 8 µM: 8, 4 and 2 µM. Naphthoquine phosphate was diluted with a 2-fold gradient into 3 concentrations 0.5 µM.: 0.5, 0.25 and 0.125 µM. The stimulant TLR4-L (final concentration: 10 µg/ml), TLR7-L (final concentration: 5 µg/ml) or TLR9-L (final concentration: 1 µM) and gradient diluted compounds were added. A corresponding stimulation control and a cell control (without stimulation) were set up. The cells were cultured in a 37°C, 5% CO₂ incubator for 48 hours. After the end of the culture, the cell culture supernatants were collected, and the levels of TNF-α, IL-1β, and IL-6 were measured by ELISA.

### (5) Antibody Level Detection

A suspension of purified mouse B cells was inoculated into a 96-well plate at 4×10⁵/well. Hydroxychloroquine sulfate was diluted with a 2-fold gradient into 3 concentrations, starting from 8 µM: 8, 4 and 2 µM. Naphthoquine phosphate was diluted with a 2-fold gradient into 3 concentrations starting from 0.5 µM.: 0.5, 0.25 and 0.125 µM. The stimulant TLR4-L (final concentration: 10 µg/ml), TLR7-L (final concentration: 5 µg/ml) or TLR9-L (final concentration: 1 µM) and gradient diluted compounds were added. A corresponding stimulation control and a cell control (without stimulation) were set up. The cells were cultured in a 37°C, 5% CO₂ incubator for 120 hours. After the end of the culture, the cell culture supernatants were collected, and the levels of IgG and IgM were measured by ELISA.

### 3.Experimental Results

As shown in Table 5, both hydroxychloroquine sulfate and naphthoquine phosphate inhibited the proliferation of purified mouse splenic B cells and the production of inflammatory cytokines and antibodies induced by specific TLR signals. The inhibitory effect of naphthoquine phosphate was superior to that of hydroxychloroquine sulfate.

**Table 5. Inhibition of the TLRs-stimulated activation of purified mouse B cells by naphthoquine phosphate in vitro**

| Compound | CC₅₀ (µM) | Stimulant | Proliferation | | IL-6 | | IgG | | IgM | |
|---|---|---|---|---|---|---|---|---|---|---|
| | | | IC₅₀ (µM) | SI | IC₅₀ (µM) | SI | IC₅₀ (µM) | SI | IC₅₀ (µM) | SI |
| Hydroxychloroquine Sulfate | 19.72 | TLR4-L | 9.95 | 1.98 | >8 | <2.47 | >8 | <2.47 | >8 | <2.47 |
| | | TLR7-L | 3.46 | 5.70 | 3.39 | 5.99 | 7.58 | 2.60 | 7.27 | 2.71 |
| | | TLR9-L | 3.29 | 5.99 | 4.03 | 4.89 | 4.54 | 4.34 | 2.01 | 9.81 |
| Naphthoquine Phosphate | 1.98 | TLR4-L | 0.19 | 10.46 | 0.5 | 3.93 | 0.41 | 4.82 | 0.21 | 9.37 |
| | | TLR7-L | 0.22 | 9.18 | 0.38 | 5.30 | >0.5 | <3.97 | >0.5 | <3.97 |
| | | TLR9-L | 0.23 | 8.69 | 0.23 | 8.59 | 0.4 | 4.91 | 0.1 | 19.03 |

| | | | | | | | | | | |
|---|---|---|---|---|---|---|---|---|---|---|
| Note: CC50 is the compound concentration at which 50% of the cells survive; IC50 is the compound concentration at which cytokine secretion is reduced by 50%; SI = CC50/IC50. | | | | | | | | | | |

Example 5. Intragastric Administration of Naphthoquine Phosphate Significantly Improved Bovine Type II Collagen-Induced Arthritis in Mice

### 1. Main Experimental Materials and Sources

### (1) Animals

SPF-grade DBA/1 mice, male, 70 in total, about 4 weeks old, purchased from Beijing Vital River Laboratory Animal Technology Co., Ltd., with certificate number 110011211101563086.

### (2) Main Experimental Agents

Naphthoquine phosphate, light yellow powder, elemental analysis (C₂₄H₃₄ClN₃O₉P₂), supplied by Shanghai Pharmaceutical Industry Co., Ltd.; hydroxychloroquine sulfate, white powder, elemental analysis (C₁₈H₂₆ClN₃O·H₂SO₄), supplied by Zhongxi Sunve Pharmaceutical Co., Ltd; and dexamethasone, purchased from Chen Xin Pharmaceutical Co., Ltd.

### (3) Reagents

Bovine type II collagen (Catalog No.: 20021), purchased from Chondrex. Inc.; acetic acid (glacial acetic acid) (Catalog No.: 10000218), purchased from Sinopharm Chemical Reagent Co., Ltd.; Freund's complete adjuvant and Freund's incomplete adjuvant, LPS, ConA, purchased from Sigma; RPMI 1640 culture medium, purchased from GibcoBRL; fetal bovine serum, purchased from Hyclone; LPS, purchased from Sigma; ConA, purchased from Sigma; purified NA/LE Hamster Anti-Mouse CD3e, purchased from BD; and HRP-rabbit anti-mouse IgG (H+L), IgG1, IgG2a, and IgG2b, purchased from Invitrogen.

### 2. Experimental Methods

### (1) Experimental Groups

Normal group, model group, positive medicament (1 mg/kg dexamethasone) group, naphthoquine phosphate (25, 50, 100 mg/kg) groups, hydroxychloroquine sulfate (100 mg/kg) group.

### (2) Model Construction

10 mg of Type II Collagen (C II) was swollen in 2.5 ml of 0.1 mol/L glacial acetic acid to prepare a 4 mg/ml C II solution. This solution was fully emulsified with an equal volume of CFA, and injected subcutaneously (50 µl) at the base of the tail of mice. After 21 days, 4 mg/ml C II solution was fully emulsified with an equal volume of IFA. This mixture was injected subcutaneously (50 µL) at the base of the tail of mice to boost the immune response.

### (3) Index Detection

### 1) Arthritis Scoring

The severity of arthritis was scored by observing joint lesions of limbs of mice. Detectable arthritis accompanied by erythema in one or more fingers was scored as 1 point, moderate redness and swelling from the ankle joint to the middle of the foot was scored as 2 points, severe redness and swelling from the ankle joint to the fingers was scored as 3 points, and severe swelling with ankylosis was scored as 4 points. During scoring, bilateral hindfoot thickness was measured.

### 2) Assessment of Lymphocyte Proliferation in Each Group by ³H-TdR Incorporation

Lymphocyte proliferation in each group was assessed using the ³H-TdR incorporation method. Lymph nodes from the spleens of mice in each group were isolated to prepare lymphocyte suspensions. The cells were inoculated into each well of a 96-well plate. ConA (final concentration: 5 µg/ml) and LPS (final concentration: 10 µg/ml) were added respectively, and cultured in a 37°C, 5% CO₂ incubator for 48 hours. Eight hours before the end of the culture, 25 µl ³H-TdR and anti-CD3 (final concentration: 5 µg/ml, pre-incubated overnight) were added to each well. The cells were cultured in a 37°C, 5% CO₂ incubator for 72 hours. C II (final concentration: 10 µg/ml) was added. The cells were cultured in a 37°C, 5% CO₂ incubator for 96 hours. Eight hours before the end of the culture, 25 µl ³H-TdR was added to each well. Corresponding cell blank controls were set up for each group. The cells were cultured until the end of the experiment. The cells were collected onto glass fiber membranes using a cell collector. 5 ml scintillation fluid was added, and the values of radioactive counts per minute were read on a Beta counter (2450 Microplate Counter, PerkinElmer).

### 3) Detection of Anti-CII Antibody Levels in Serum by ELISA

50 µl C II solution (final concentration: 50 µg/ml) was added to each well of a 96-well ELISA plate for coating overnight. The plate was washed 4 times with 200 µl washing solution (1L PBS + 500 µl Tween 20) per well. 100 µl 1% BSA was added to each well to block for 1 hour. The plate was then washed 4 times with 200 µl washing solution per well. 50 µl diluted serum samples from each group of mice were added to each well and incubated for 2 hours. The plate was washed 4 times with 200 µl washing solution per well. Horseradish Peroxidase (HRP)-conjugated IgG secondary antibody was added and incubated for 1 hour. The plate was washed 5 times with 200 µl washing solution per well. 50 µl tetramethylbenzidine (TMB) substrate was added, and 30 µl of 1M sulfuric acid solution was added to each well to terminate color development based on the condition of color development. Absorbance values at 450 nm/570 nm were read by a microplate reader (Spectra Max 190, Molecular Devices).

### 4) Bone Morphological Analysis by Micro-Computed Tomography (Micro-CT)

Three-dimensional reconstruction was performed by the micro-CT technology, and bone morphology analysis was performed by professional analysis software.

### 3. Experimental Results

### (1) Intragastric Administration of Naphthoquine Phosphate Significantly Improved Clinical Scores and Paw Swelling in Arthritic Mice

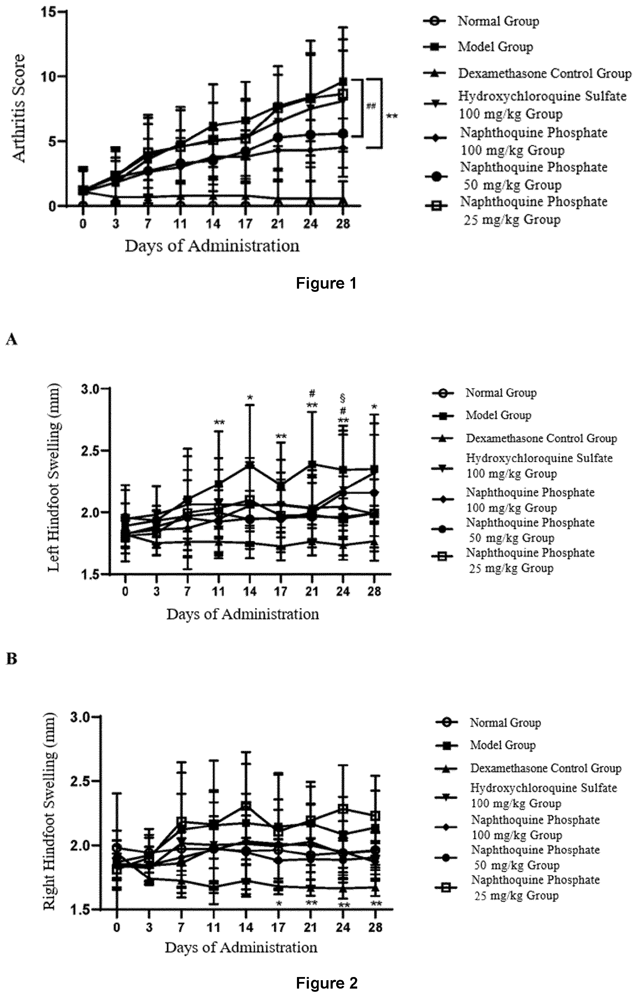

As shown in Figure 1, mice in the model group developed severe joint lesions. Administration of naphthoquine phosphate significantly reduced the arthritis scores of model mice, and the therapeutic effect was superior to that of hydroxychloroquine sulfate at the same dose. (Note: *P value <0.05, **P value <0.01, ***P value <0.001, 100 mg/kg naphthoquine phosphate compared with model group; # P value <0.05, ## P value <0.01, ### P value <0.001, 50 mg/kg naphthoquine phosphate compared with model group.)

As shown in Figure 2, administration of naphthoquine phosphate improved foot swelling in arthritic mice. (Note: *P value <0.05, **P value <0.01, ***P value <0.001, dexamethasone control group compared with model group; # P value <0.05, ## P value <0.01, ### P value <0.001, normal group compared with model group; § P value <0.05, §§ P value <0.01, §§§ P value <0.001, 25 mg/kg naphthoquine phosphate group compared with model group.)

### (2) Intragastric Administration of Naphthoquine Phosphate Significantly Inhibited Proliferation of Spleen and Lymph Node Lymphocytes in Arthritic Mice

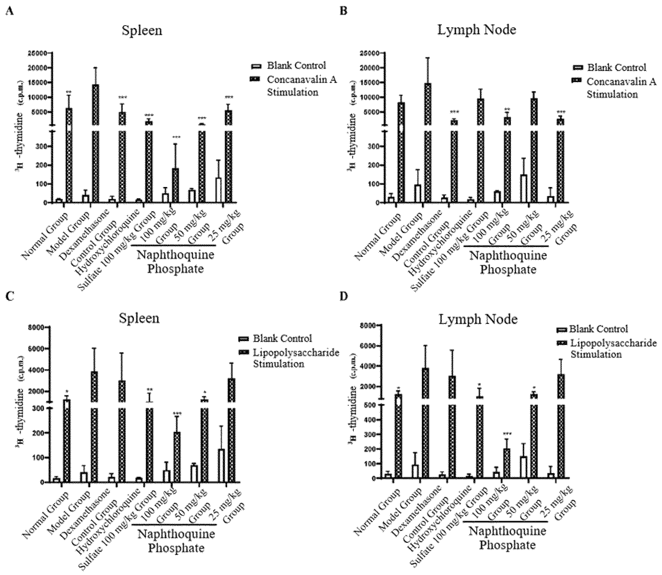

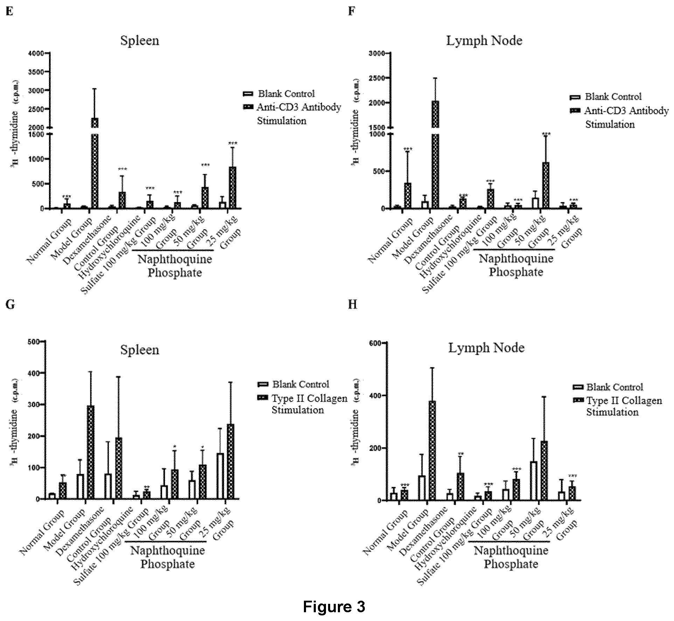

As shown in Figure 3, at the end of the experiment, mouse spleen lymph nodes were removed to prepare single cell suspensions, and different stimulants were used to stimulate their proliferation. The results shows that administration of naphthoquine phosphate can significantly inhibit proliferation of spleen and lymph node lymphocytes in arthritic mice. (Note: *P value<0.05, **P value<0.01, ***P value<0.001, compared with model group.)

### (3) Intragastric Administration of Naphthoquine Phosphate Significantly Reduced the Levels of Collagen-Specific Antibodies in Arthritic Mice

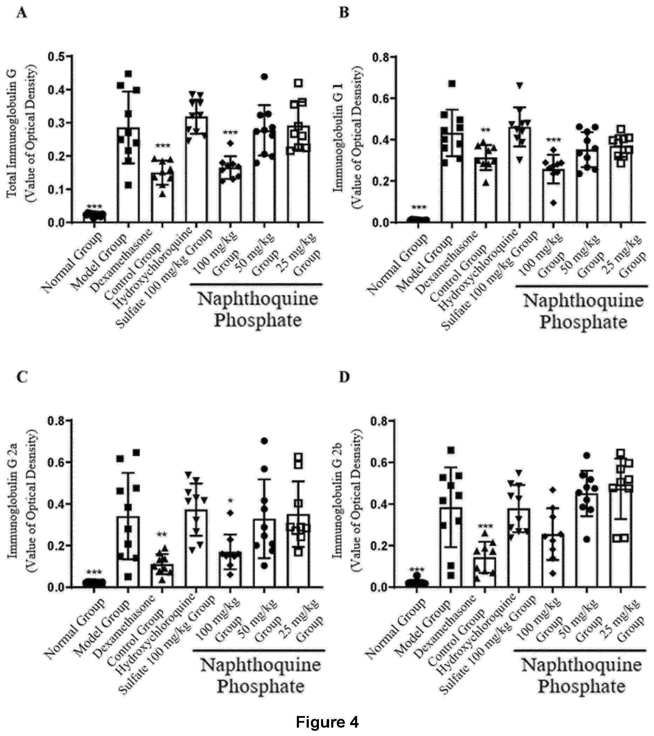

As shown in Figure 4, at the end of the experiment, eyeballs were removed to collect blood, and serum was separated. ELISA method was used to detect the levels of collagen-specific antibodies in serum of experimental mice. The results shows that the administration of naphthoquine phosphate can reduce the levels of collagen-specific antibodies in serum of arthritic mice. (Note: *P value<0.05, **P value<0.01, ***P value<0.001, compared with model group.)

### (4) Intragastric Administration of Naphthoquine Phosphate Significantly Improved Bone Damage Caused by Bone Erosion in Joints of Arthritic Mice

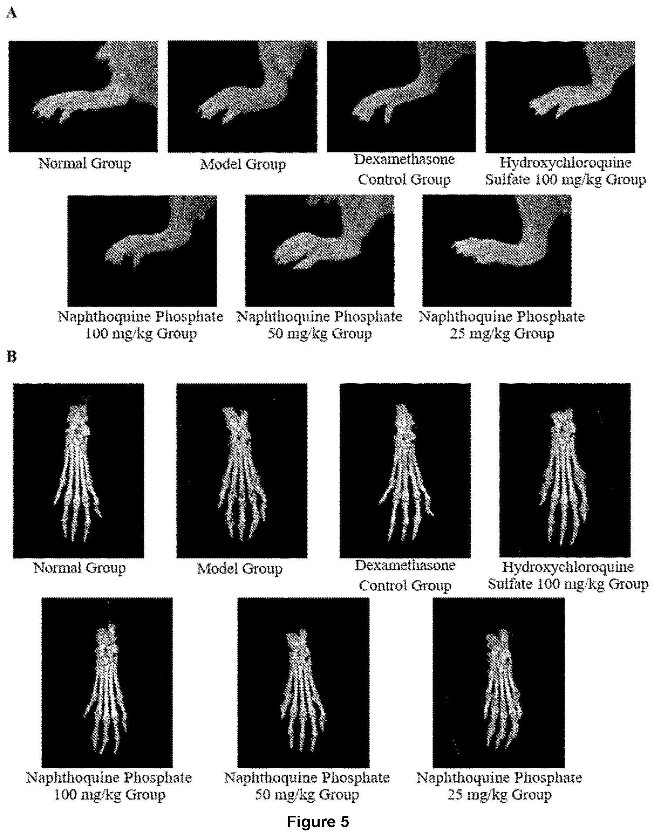

At the end of the experiment, photographs of the hind limbs of mice in each group were taken. The left hind limb was separated and subjected to Micro-CT scanning and analysis. As shown in Figure 5, mice in the model group shows obvious joint swelling and deformation, with severe bone erosion. Administration of naphthoquine phosphate inhibited bone damage in model mice, demonstrating a certain degree of bone-protection effect.

Example 6. Intragastric Administration of Naphthoquine Phosphate Significantly Inhibited the Activation of Purified Splenic B Cells Induced by TLRs-L in Arthritic Mice

### 1. Main Experimental Materials and Sources

### (1) Animals

SPF-grade DBA/1 mice, males, 70 in total, about 4 weeks old, purchased from Beijing HFK Bioscience Co., Ltd., with certificate number 110322211101922685.

### (2) Main Experimental Agents

Naphthoquine phosphate, light yellow powder, elemental analysis (C₂₄H₃₄ClN₃O₉P₂), supplied by Shanghai Pharmaceutical Industry Co., Ltd.; hydroxychloroquine sulfate, white powder, elemental analysis (C₁₈H₂₆ClN₃O·H₂SO₄), supplied by Zhongxi Sunve Pharmaceutical Co., Ltd.

### (3) Reagents

Bovine type II collagen (Catalog No.: 20021), purchased from Chondrex. Inc.; acetic acid (glacial acetic acid) (Catalog No.: 10000218), purchased from Sinopharm Chemical Reagent Co., Ltd.; Freund's complete adjuvant and Freund's incomplete adjuvant, purchased from Sigma; RPMI 1640 culture medium, purchased from GibcoBRL; fetal bovine serum, purchased from Hyclone; Rat Anti-Mouse CD16/CD32 (2.4G2), PE Rat Anti-Mouse CD19 and mouse IL-6 ELISA detection kits, purchased from BD; Anti-PE Beads, MACS column, and MACS buffer, purchased from Miltenyi; TLRs- L, purchased from Invivogen; antibody detection kit, purchased from Invitrogen; and flow detection antibodies, purchased from BD. ³H-TdR and scintillation fluid were purchased from PerkinElmer.

### 2. Experimental Methods

### (1) Experimental Groups

Normal group, model group, positive medicament (1mg/kg methotrexate) group, naphthoquine phosphate (50, 100 mg/kg) group, hydroxychloroquine sulfate (100 mg/kg) group.

### (2) Model construction

Same as those described in Example 5.

### (3) Index Detection

At the end of the experiment, spleens of mice in each group were collected, and splenic B cells were purified as described in Example 4. Different TLRs-L were used for stimulation. ³H-TDR incorporation method was used to detect proliferation, ELISA method was used to detect the levels of IL-6 and antibody secretion, and flow cytometry was used to detect the expressions of CD80, CD86, and CD69.

### 1) B Cell Proliferation Assay

Suspensions of purified mouse B cells from each group were inoculated into a 96-well plate at 4×10⁵/well, and the stimulant TLR4-L (final concentration: 10 µg/ml), TLR7-L (final concentration: 5 µg/ml) or TLR9-L (final concentration: 1 µM) was added. Corresponding blank controls were set up for each group. The cells were cultured in a 37°C, 5% CO₂ incubator for 48 hours. Eight hours before the end of the culture, ³H-TdR was added, and the culture was continued until the end of the experiment. The cells were collected onto glass fiber membranes using a cell collector. 5 ml scintillation fluid was added, and the values of radioactive counts per minute were read on a Beta counter (2450 Microplate Counter, PerkinElmer).

### 2) Cytokine Detection

Suspensions of purified mouse B cells from each group were inoculated into a 96-well plate at 4×10⁵/well, and the stimulant TLR4-L (final concentration: 10 µg/ml), TLR7-L (final concentration: 5 µg/ml) or TLR9-L (final concentration: 1 µM) was added. Corresponding blank controls were set up for each group. The cells were cultured in a 37°C, 5% CO₂ incubator for 48 hours. After the end of the culture, the cell culture supernatants were collected, and the level of IL-6 was measured by ELISA.

### 3) Antibody Level Detection

A suspension of purified mouse B cells was inoculated into a 96-well plate at 4×10⁵/well, and the stimulant TLR4-L (final concentration: 10 µg/ml), TLR7-L (final concentration: 5 µg/ml) or TLR9-L (final concentration: 1 µg/ml) was added. Corresponding blank controls were set up for each group. The cells were cultured in a 37°C, 5% CO₂ incubator for 120 hours. After the end of the culture, the cell culture supernatants were collected, and the levels of IgG and IgM were measured by ELISA.

### 4) Detection of CD86, CD80, and CD69 Expression by Flow Cytometry

Suspension of purified mouse B cells from each group was inoculated into a 96-well plate at 4×10⁵/well, and the stimulant TLR4-L (final concentration: 10 µg/ml), TLR7-L (final concentration: 5 µg/ml) or TLR9-L (final concentration: 1 µM) was added. Corresponding blank controls were set up for each group. The cells were cultured in a 37°C, 5% CO₂ incubator for 48 hours. The cells were collected into 4 ml FACS washing solution (formula) and centrifuged at 4°C, 500 G for 6 min. 5 µl 2.4G2 was added to each tube and blocked for 20 min, and shaked once every 10 min. 10 µl flow cytometry antibody dye was added to each tube and incubated for 20 min, shaked once every 10 min. 4 ml FACS washing solution was added to each tube, and centrifuged at 4°C, 500G for 6 min. 300 µl FACS washing solution was added to each tube to resuspend the cells, and the expressions of CD86, of CD80 and CD69 were detected by a flow cytometer (LSR FORTESSA, BD).

### 3. Experimental Results

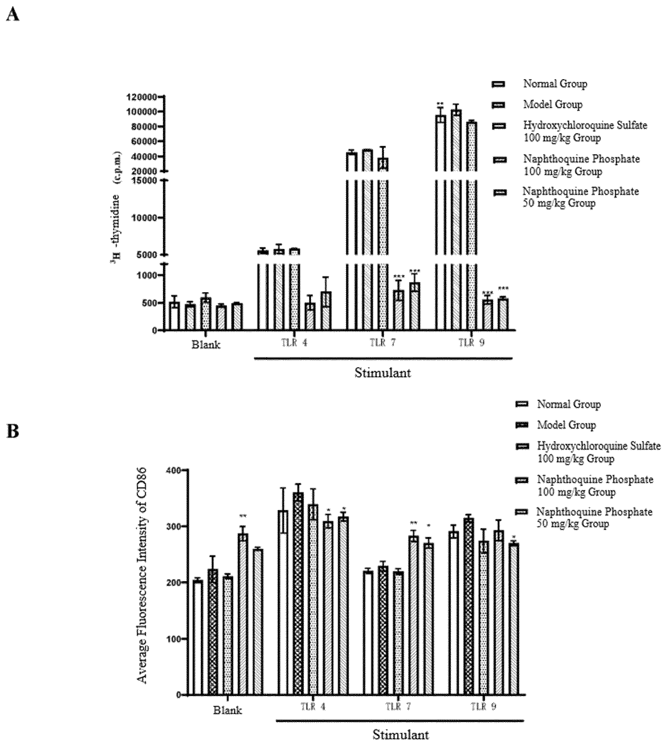

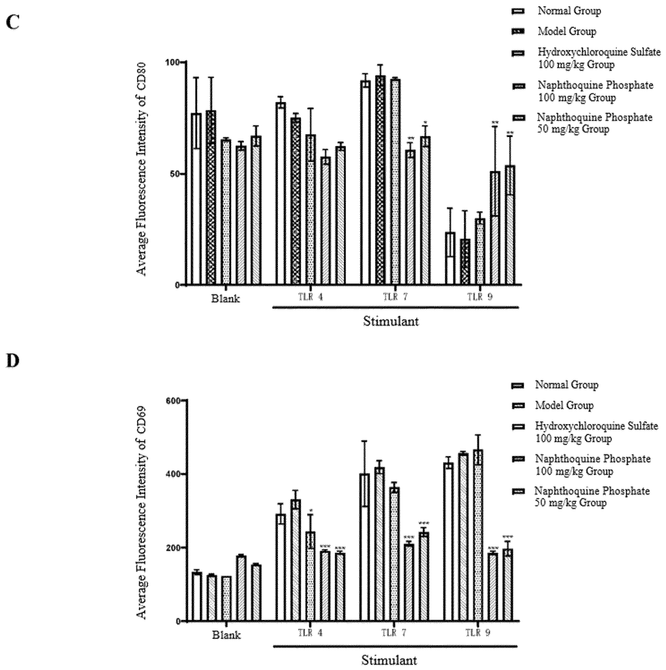

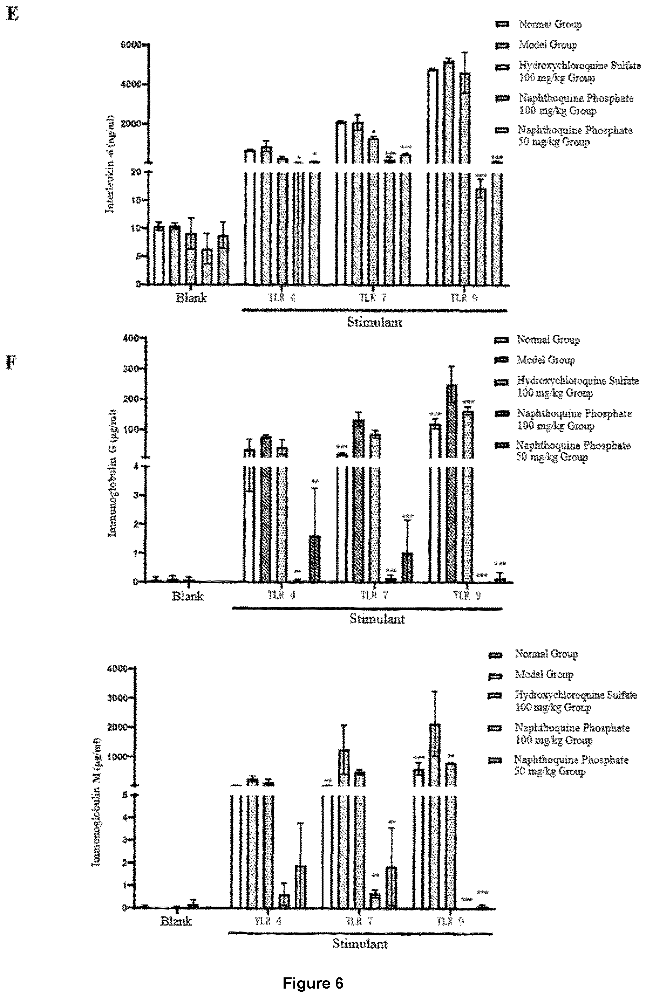

As shown in Figure 6, administration of naphthoquine phosphate significantly inhibited B cell activation, cytokine secretion and antibody production mediated by TLR signaling, suggesting that it may inhibit the pathological immune response of B cells in RA by blocking TLR signaling. (Note: *P value<0.05, **P value<0.01, ***P value<0.001, compared with model group.)

Example 7. Intragastric Administration of Naphthoquine Phosphate Significantly Improved Disease Indicators of Adjuvant-Induced Arthritis in Rats

### 1. Main Experimental Materials and Sources

### (1) Animals

SD rats, male, 38 in total, about 150 g, purchased from Beijing HFK Bioscience Co., Ltd., with certificate number 110322211101936668.

### (2) Main Experimental Agents

Naphthoquine phosphate, light yellow powder, elemental analysis (C₂₄H₃₄ClN₃O₉P₂), supplied by Shanghai Pharmaceutical Industry Co., Ltd.; hydroxychloroquine sulfate, white powder, elemental analysis (C₁₈H₂₆ClN₃O·H₂SO₄), supplied by Zhongxi Sunve Pharmaceutical Co., Ltd.; methotrexate for injection, purchased from Furen Pharmaceutical Co., Ltd.

### (3) Reagents

BCG vaccine, purchased from DIFCO; lanolin, purchased from Sinopharm Chemical Reagent Co., Ltd.; and paraffin oil, purchased from Sinopharm Chemical Reagent Co., Ltd.

### 2. Experimental Methods

### (1) Experimental Groups

Normal group, model group, positive medicament (1mg/kg methotrexate) group, naphthoquine phosphate (25, 50, 100 mg/kg) group, hydroxychloroquine sulfate (100 mg/kg) group.

### (2) Model construction

An appropriate amount of lanolin and paraffin oil was sterilized and placed on ice for later use. One vial of BCG vaccine (100 mg) + 1.5 ml PBS were grinded with a glass homogenizer until no visible particles remain. After thorough grinding, 3.5 ml PBS was added and placed on ice for later use. The sterilized lanolin was heated in a 56°C water bath until it becomes liquid. 675 µl paraffin oil, 225 µl lanolin and 900 µl thoroughly ground BCG vaccine-containing PBS were dispensed in an emulsification tube and emulsified thoroughly. 0.1 ml of the emulsion was injected intradermally into the footpad of the left hindfoot of the rat.

### (3) Index Detection

### 1) Arthritis Scoring

The severity of arthritis was scored by observing the secondary lesions of foot joints in rats. Detectable arthritis accompanied by erythema in one or more fingers was scored as 1 point, moderate redness and swelling from the ankle joint to the middle of the foot was scored as 2 points, severe redness and swelling from the ankle joint to the fingers was scored as 3 points, and severe swelling with ankylosis was scored as 4 points. During scoring, bilateral hindfoot thickness was measured.

### 2) Pathological Analysis of Joints in Model Animals

Bone and joint tissues were analyzed by H&E staining.

### 3) Bone Morphological Analysis by Micro-Computed Tomography (Micro-CT)

Three-dimensional reconstruction was performed by the micro-CT technology, and bone morphology analysis was performed by professional analysis software.

### 3. Experimental Results

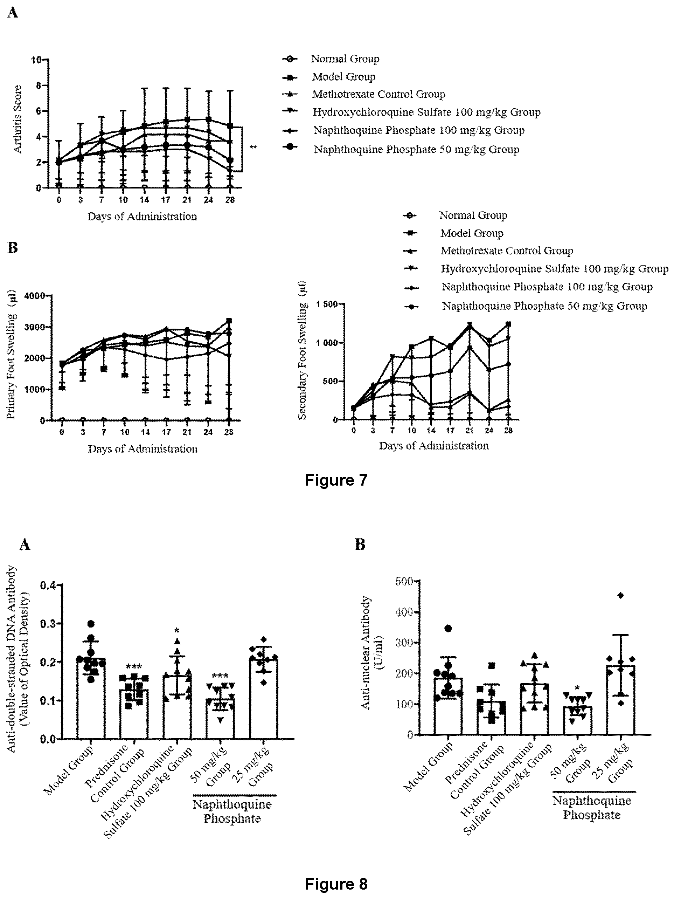

As shown in Figure 7, rats in the model construction group suffered from severe swelling and deformation in the ankle joints and toe joints. Administration of naphthoquine phosphate improved the clinical arthritis scores of the experimental rats and inhibited the extent of foot swelling. The therapeutic effect was superior to that of hydroxychloroquine sulfate at the same dose. (Note: *P value<0.05, **P value<0.01, ***P value<0.001, compared with model group.)

Example 8. Intragastric Administration of Naphthoquine Phosphate Significantly Reduced Spontaneous Systemic Lupus Erythematosus in Experimental Mice

### 1. Main Experimental Materials and Sources

### (1) Animals

SPF-grade MRL/lpr mice, female, about 6-8 weeks old, purchased from Shanghai Lingchang Biotechnology Co., Ltd., with certificate number 20180003016877.

### (2) Main Experimental Agents

Naphthoquine phosphate, light yellow powder, elemental analysis (C₂₄H₃₄ClN₃O₉P₂), supplied by Shanghai Pharmaceutical Industry Co., Ltd.; hydroxychloroquine sulfate, white powder, elemental analysis (C₁₈H₂₆ClN₃O·H₂SO₄), supplied by Zhongxi Sunve Pharmaceutical Co., Ltd.; prednisone, purchased from Shanghai Jinsui Biotechnology Co., Ltd.

### (3) Reagents

Coomassie brilliant blue staining solution, purchased from BIO-RAD; creatinine detection kit, purchased from Abcam; mouse anti-nuclear antibody (ANA) kit, purchased from Alpha Diagnostic International Co., Ltd.; HRP-labeled goat anti-mouse IgG (H+L), purchased from Beyotime; and calf thymus DNA, purchased from Sigma.

### 2. Experimental Methods

### (1) Experimental Groups

Normal group, model group, positive medicament (1 mg/kg prednisone) group, naphthoquine phosphate (25, 50 mg/kg) group, hydroxychloroquine sulfate (100 mg/kg) group.

### (2) Proteinuria and Urinary Creatinine Measurement

After weighing the mice, 20 µl urine was collected for each mouse. The protein content in the urine samples was determined using Coomassie Brilliant Blue, and urine creatinine level was determined using a creatinine kit.

### (3) Detection of Anti-Double-Stranded DNA Antibodies

50 µl double-stranded DNA solution (final concentration: 50 µg/ml) was added to each well of a 96-well ELISA plate for coating overnight. The plate was washed 4 times with 200 µl washing solution (1L PBS+500 µl Tween 20) per well. 100 µl 1% BSA was added to each well and blocked for 1 hour. The plate was washed 4 times with 200 µl washing solution per well. 50 µl diluted mouse serum samples of each group were added to each well and incubated for 2 hours. The plate was washed 4 times with 200 µl washing solution per well. Horseradish peroxidase (HRP)-conjugated IgG (H+L) secondary antibodies were added and incubated for 1 hour. The plate was washed 5 times with 200 µl washing solution per well. 50 µl tetramethylbenzidine (TMB) substrate was added, and 30 µl 1M sulfuric acid solution was added to terminate color development based on the condition of color development. Absorbance values at 450nm/570nm were read by a microplate reader (Spectra Max 190, Molecular Devices).

### 3. Experimental Results

At the end of the experiment, eyeballs were removed to collect blood. ELISA method was used to detect the levels of anti-dsDNA antibodies in serum of experimental mice, and the anti-nuclear antibody kit was used to detect the levels of anti-nuclear antibodies. As shown in Figure 8, administration of naphthoquine phosphate significantly reduced the levels of anti-dsDNA antibodies and anti-nuclear antibodies in serum of experimental mice. (Note: *P value<0.05, **P value<0.01, ***P value<0.001, compared with model group.)

The above results show that the administration of naphthoquine phosphate can significantly improve disease indicators in arthritic mice, reduce the levels of collagen-specific antibodies in serum, and inhibit proliferation of lymphocytes; significantly improve the clinical scores of arthritic rats, inhibit secondary foot swelling; and reduce the levels of pathogenic antibodies in serum of lupus model mice; inhibit activation of B cells mediated by TLR signaling, thereby exerting a therapeutic effect on autoimmune diseases, and can be developed as a therapeutic medicament for autoimmune diseases.

### References

[1] Chang Y J, Liu K S, Wang J J, et al. Antimalarial primaquine for skin infiltration analgesia in rats [J]. J Pharm Pharmacol, 2021, 73(2): 206-11.
[2] Tsokos G C. Systemic lupus erythematosus [J]. N Engl J Med, 2011, 365(22): 2110-21.
[3] Lee S J, Silverman E, Bargman J M. The role of antimalarial agents in the treatment of SLE and lupus nephritis [J]. Nat Rev Nephrol, 2011,7(12): 718-29.

## Claims

1. Use of naphthoquine phosphate in preparation of a medicament for treating an autoimmune disease.

2. The use according to claim 1, wherein the autoimmune disease is rheumatoid arthritis or systemic lupus erythematosus.

3. The use according to claim 1, wherein the use is use of naphthoquine phosphate in preparation of a medicament for treating or auxiliary treating rheumatoid arthritis.

4. The use according to claim 1, wherein the use is use of naphthoquine phosphate in preparation of a medicament for treating or auxiliary treating systemic lupus erythematosus.

5. The use according to claim 1, 3 or 4, wherein the medicament is a pharmaceutical composition consisting of a therapeutically effective amount of naphthoquine phosphate as an active ingredient and pharmaceutical excipient(s).

6. The use according to claim 5, wherein the pharmaceutical composition is in an oral or injectable dosage form.

7. The use according to claim 6, wherein the oral dosage form is a tablet, capsule, granule or oral liquid preparation; and wherein the injectable dosage form is an intravenous or intramuscular injection.
